# EUROPEAN PATENT APPLICATION

(11) **EP 4 818 704 A1**
(43) Date of publication of application: **07.10.2026**
(21) Application number: 24912755.6
(22) Date of filing: 23.12.2024
(51) Int. Cl.: G01P 15/00, G01P 15/18

(54) **IMPACT DETECTION DEVICE, ELECTRONIC APPARATUS, IMPACT DETECTION METHOD, AND IMPACT DETECTION PROGRAM**

(30) Priority: 26.12.2023 JP 2023219618
(71) Applicant: Terumo Kabushiki Kaisha, Tokyo 151-0072 (JP)
(72) Inventor: TAGAMI, Toshihiro, Ashigarakami-gun, Kanagawa 259-0151 (JP); SUZUKI, Yasuo, Ashigarakami-gun, Kanagawa 259-0151 (JP)
(74) Representative: KIPA AB
(86) International application number: PCT/JP2024/045535
(87) International publication number: WO 2025/142868

(57) **Abstract**

An impact detection device includes: a communication unit that communicates with an acceleration sensor included **in** an electronic device; and a control unit that refers to a notification signal, upon receiving a drop signal indicating that the electronic device has dropped, from the acceleration sensor via the communication unit, the notification signal being received from the acceleration sensor via the communication unit within a certain time period following reception of the drop signal, that determines whether the electronic device has received an impact resulting from an unpackaged drop of the electronic device from a specific height or higher, and that performs recording at least upon being determined that the electronic device has received the impact.

## Description

### Technical Field

The present disclosure relates to an impact detection device, an electronic device, an impact detection method, and an impact detection program.

### Background Art

Patent Literature 1 discloses a device that calculates a time integral of absolute-value waveforms of three-axis acceleration values, once in every predetermined time period, the three-axis accelerations being detected by a triaxial acceleration sensor, and that detects the impact when the voltage resulting from the integration is higher than a threshold. Patent Literature 2 discloses an electronic device that calculates a second threshold, when it is determined that the acceleration value of an acceleration detected at a predetermined time interval exceeds a first threshold, the second threshold being calculated on the basis of a maximum acceleration value, among the acceleration values of a plurality of respective accelerations detected over a predetermined time period subsequent to the determination, and determines that multiple impacts have occurred when the number of accelerations having acceleration values exceeding the second threshold is more than a predetermined number. Patent Literature 3 discloses a device that detects an impact applied to a package.

### Citation List

### Patent Literature

Patent Literature 1: JP 2008-020250 A
Patent Literature 2: JP 2017-054330 A
Patent Literature 3: JP 2017-156236 A

### Summary of Invention

### Technical Problem

A pump such as a syringe pump or an infusion pump has an impact detection function that uses an acceleration sensor. For example, when the pump receives an impact resulting from an unpackaged drop of the pump from one meter, a detection of the impact is recorded in an operation history, and a user is notified that the function of the product may have been impaired by the impact.

There are two types of acceleration sensors in terms of their applications: namely, low G acceleration sensors and high G acceleration sensors. Low G acceleration sensors can detect very small changes such as changes in walking or body movements, but are not suitable for detecting large changes such as an impact. High G acceleration sensors can detect a large change such as an impact, but are not suitable for detecting very small changes. In order to detect both very small and large changes, a low G acceleration sensor and a high G acceleration sensor may be both incorporated into a pump, but it leads to an increase in cost. To comply with RoHS, it may be necessary to use only a low G acceleration sensor, which is not suitable for impact detection. "RoHS" is an abbreviation for Restriction of Hazardous Substances Directive.

Even if a pump drops from a specific height, such as one meter, as long as the pump is in a package, the function of the product is less likely to be impaired by the impact. Therefore, it is desirable to make a distinction between a large impact resulting from an unpackaged drop of a pump from a specific height or higher, and a small impact resulting from a drop of the pump from a lower position or resulting from a packaged drop of the pump.

An object of the present disclosure is to enable detection of a large impact resulting from an unpackaged drop of the electronic device from a specific height or higher, in a manner distinguished from a small impact.

### Solution to Problem

Some aspects of the present disclosure will be described below.
[1] An impact detection device including:
   a communication unit that communicates with an acceleration sensor included in an electronic device; and
   a control unit that refers to a notification signal, upon receiving a drop signal indicating that the electronic device has dropped, from the acceleration sensor via the communication unit, the notification signal being received from the acceleration sensor via the communication unit within a certain time period following the reception of the drop signal, that determines whether the electronic device has received an impact resulting from an unpackaged drop of the electronic device from a specific height or higher, and that performs recording at least upon being determined that the electronic device has received the impact.
[2] The impact detection device according to [1], in which the control unit receives time-series signals of three-axis accelerations, as the notification signal, applies high-pass filtering processing to the time-series signals, and determines whether the electronic device has received the impact based on whether a sum of resultant vectors of the three-axis accelerations after the high-pass filtering processing is equal to or greater than a threshold.
[3] The impact detection device according to [1], in which the control unit increments a counter every time a signal indicating that an acceleration of a magnitude exceeding a reference is measured is received as the notification signal, and determines whether the electronic device has received the impact based on whether a value of the counter has exceeded a threshold within the certain time period.
[4] The impact detection device according to any one of [1] to [3], in which the drop signal is a time-series signal of three-axis acceleration, and is a signal in which the acceleration in each axis continuously takes a value within a certain range less than 1 G and around 0 G over a reference time period or longer.
[5] The impact detection device according to any one of [1] to [4], in which, when the control unit determines that the electronic device has received the impact, the control unit notifies a user of presence of an unpackaged drop of the electronic device from the specific height or higher.
[6] An electronic device including:
   the impact detection device according to any one of [1] to [5]; and
   the acceleration sensor.
[7] The electronic device according to [6], wherein the electronic device is a medical device.
[8] An impact detection method in which a computer that communicates with an acceleration sensor provided in an electronic device executes:
   referring to a notification signal, upon receiving a drop signal indicating that the electronic device has dropped, from the acceleration sensor, the notification signal being received from the acceleration sensor within a certain time period following reception of the drop signal, and determining whether the electronic device has received an impact resulting from an unpackaged drop of the electronic device from a specific height or higher; and
   performing recording at least in a case where the computer determines that the electronic device has received the impact.
[9] An impact detection program for causing a computer that communicates with an acceleration sensor provided in an electronic device to execute an operation including:
   referring to a notification signal, upon receiving a drop signal indicating that the electronic device has dropped, from the acceleration sensor, the notification signal being received from the acceleration sensor within a certain time period following reception of the drop signal, and determining whether the electronic device has received an impact resulting from an unpackaged drop of the electronic device from a specific height or higher; and
   performing recording at least upon being determined that the electronic device has received the impact.

### Advantageous Effects of Invention

According to the present disclosure, it is possible to detect a large impact resulting from an unpackaged drop of the electronic device from a specific height or higher, in a manner distinguished from a small impact.

### Brief Description of Drawings

[Fig. 1] Fig. 1 is a block diagram illustrating a configuration of an electronic device according to an embodiment of the present disclosure.
[Fig. 2] Fig. 2 is a perspective view of the electronic device according to the embodiment of the present disclosure.
[Fig. 3] Fig. 3 is a flowchart illustrating an operation of an impact detection device according to the embodiment of the present disclosure.
[Fig. 4] Fig. 4 is a diagram illustrating an example of the waveforms of three-axis accelerations during a drop.
[Fig. 5] Fig. 5 is a diagram illustrating an example of frequency spectrums of accelerations without packaging and with packaging.
[Fig. 6] Fig. 6 is a flowchart illustrating a modification of the operation illustrated in Fig. 3.

### Description of Embodiments

An embodiment of the present disclosure will now be described with reference to drawings.

In the drawings, the same or corresponding parts are denoted by the same reference numerals. In the description of this embodiment, description of the same or corresponding parts will be omitted or simplified as appropriate.

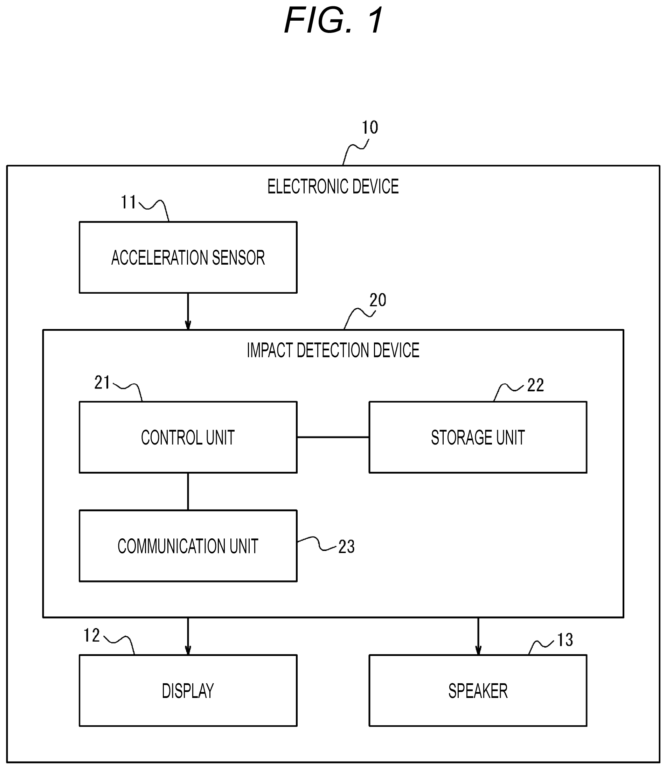

A configuration of an electronic device 10 according to this embodiment will now be described with reference to Fig. 1.

The electronic device 10 includes an acceleration sensor 11 and an impact detection device 20. In this embodiment, the electronic device 10 further includes a display 12 and a speaker 13, which are not essential.

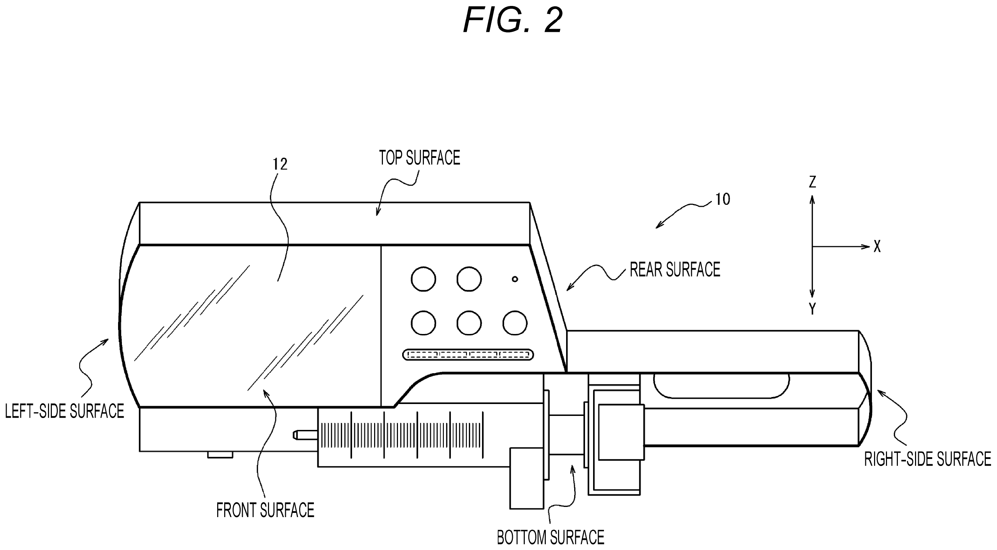

The electronic device 10 is a medical device. In this embodiment, as illustrated in Fig. 2, the electronic device 10 is a syringe pump that delivers a medication filled in a syringe by pressing a syringe plunger of the syringe, but may also be an infusion pump that delivers a medication flowing through an infusion tube by sequentially pressing the infusion tube, or another type of medical device. Alternatively, the electronic device 10 may be mobile equipment other than a medical device, such as a smartphone, a tablet, a laptop PC, or a portable gaming machine. "PC" is an abbreviation for personal computer.

The acceleration sensor 11 is a sensor that measures accelerations in three axes, namely, an X axis, a Y axis orthogonal to the X axis, and a Z axis orthogonal to the X axis and orthogonal to the Y axis. As illustrated in Fig. 2, the X axis is an axis extending in the length direction of the electronic device 10, the Y axis is an axis extending in the width direction of the electronic device 10, and the Z axis is an axis extending in the height direction of the electronic device 10. In this embodiment, the acceleration sensor 11 is a low G acceleration sensor having a measurement range of about 8 G or below.

The display 12 is a device that displays various types of information related to the electronic device 10 on a screen. The display 12 is, for example, an LCD or an organic EL display. "LCD" is an abbreviation for liquid crystal display. "EL" is an abbreviation for electro luminescent.

The speaker 13 is a device that outputs various types of information related to the electronic device 10 via voice.

In this embodiment, the impact detection device 20 is mounted on the electronic device 10, but may be installed separately from the electronic device 10 and communicate with the electronic device 10 directly or via a network such as a LAN or the Internet. "LAN" is an abbreviation for local area network. The impact detection device 20 is, for example, a microcomputer mounted on the electronic device 10, but may be a general-purpose computer such as a PC, a server computer such as a cloud server, a mobile device such as a smartphone or a tablet, or a dedicated computer, each installed separately from the electronic device 10.

An outline of this embodiment will now be described.

Upon receiving a drop signal indicating that the electronic device 10 has dropped, from the acceleration sensor 11, the impact detection device 20 refers to a notification signal received from the acceleration sensor 11 within a certain time period following reception of the drop signal, and determines whether the electronic device 10 has received an impact resulting from an unpackaged drop from a specific height or higher. The impact detection device 20 performs recording at least in a case where it is determined that the electronic device 10 has received the impact.

According to this embodiment, it is possible to detect a large impact resulting from an unpackaged drop of the electronic device 10 from a specific height or higher, in a manner distinguished from a small impact.

A configuration of the impact detection device 20 according to this embodiment will be described with reference to Fig. 1.

The impact detection device 20 includes a control unit 21, a storage unit 22, and a communication unit 23.

The control unit 21 includes at least one processor, at least one programmable circuit, at least one dedicated circuit, or any combination thereof. The processor is a general-purpose processor such as a CPU or GPU, or a dedicated processor specialized for specific processing. "CPU" is an abbreviation for central processing unit. "GPU" is an abbreviation for graphics processing unit. The programmable circuit is, for example, an FPGA. "FPGA" is an abbreviation for field-programmable gate array. The dedicated circuit is, for example, an ASIC. "ASIC" is an abbreviation for application specific integrated circuit. The control unit 21 executes processing related to an operation of the impact detection device 20 while controlling each unit included in the impact detection device 20.

The storage unit 22 includes at least one semiconductor memory, at least one magnetic memory, at least one optical memory, or any combination thereof. The semiconductor memory is, for example, a RAM, a ROM, or a flash memory. "RAM" is an abbreviation for random access memory. "ROM" is an abbreviation for read only memory. The RAM is, for example, an SRAM or a DRAM. "SRAM" is an abbreviation for static random access memory. "DRAM" is an abbreviation for dynamic random access memory. The ROM is, for example, an EEPROM. "EEPROM" is an abbreviation for electrically erasable programmable read only memory. The flash memory is, for example, an SSD. "SSD" is an abbreviation for solid-state drive. The magnetic memory is, for example, an HDD. "HDD" is an abbreviation for a hard disk drive. The storage unit 22 functions as, for example, a main storage device, an auxiliary storage device, or a cache memory. The storage unit 22 stores data to be used for the operation of the impact detection device 20 and data obtained as a result of the operation of the impact detection device 20.

The communication unit 23 includes at least one communication module. The communication module is a module compatible with, for example, a serial communication standard such as SPI or I2C, a wired LAN communication standard such as Ethernet (registered trademark), a wireless LAN communication standard such as IEEE 802.11, a mobile communication standard such as LTE, a 4G standard, or a 5G standard, or a near field wireless communication standard such as Bluetooth (registered trademark). "SPI" is an abbreviation for Serial Peripheral Interface. "I2C" is an abbreviation for Inter-Integrated Circuit. "IEEE" is an abbreviation for Institute of Electrical and Electronics Engineers. "LTE" is an abbreviation for Long Term Evolution. "4G" is an abbreviation for 4th generation. "5G" is an abbreviation for 5th generation. The communication unit 23 communicates with the acceleration sensor 11. Specifically, the communication unit 23 communicates with the acceleration sensor 11 via serial communication such as SPI or I2C. The communication unit 23 may communicate with the display 12. The communication unit 23 may communicate with the speaker 13. The communication unit 23 receives data to be used for the operation of the impact detection device 20, and transmits data obtained as a result of the operation of the impact detection device 20.

The function of the impact detection device 20 is implemented by causing a processor serving as the control unit 21 to execute the impact detection program according to this embodiment. That is, the function of the impact detection device 20 is implemented by software. The impact detection program causes a computer to function as the impact detection device 20 by causing the computer to execute an operation of the impact detection device 20. In other words, a computer functions as the impact detection device 20, by executing the operation of the impact detection device 20 in accordance with an impact detection program.

The program may be stored in a non-transitory computer-readable medium. The non-transitory computer-readable medium is, for example, a flash memory, a magnetic recording device, an optical disc, a magneto-optical recording medium, or a ROM. The program is distributed by, for example, selling, transferring, or lending a portable medium such as an SD card, a DVD, or a CD-ROM that stores the program. "SD" is an abbreviation for Secure Digital. "DVD" is an abbreviation for digital versatile disc. "CD-ROM" is an abbreviation for compact disc read only memory. The program may also be distributed by storing the program in a storage of a server and causing the server to transmit the program to another computer. The program may be provided as a program product.

The computer temporarily stores, for example, a program stored in the portable medium or a program transferred from the server, in the main storage device. The computer causes the processor to read the program stored in the main storage device, and to execute processing in accordance with the read program. The computer may also read the program directly from the portable medium, and execute the processing in accordance with the program. Every time a program is transferred from the server to the computer, the computer may sequentially execute processing in accordance with the received program. The processing may be executed by what is generally known as an ASP service in which a function is implemented solely by giving instructions of execution and acquiring the results, without transferring a program from the server to the computer. "ASP" is an abbreviation for application service provider. The program includes information equivalent to a program, the information being intended for the use in processing executed by an electronic computer. For example, "information equivalent to a program" includes data having a property that determines the processing of a computer, despite not being a direct command to a computer.

Some or all of the functions of the impact detection device 20 may be implemented by a programmable circuit or a dedicated circuit serving as the control unit 21. That is, some or all of the functions of the impact detection device 20 may be implemented by hardware.

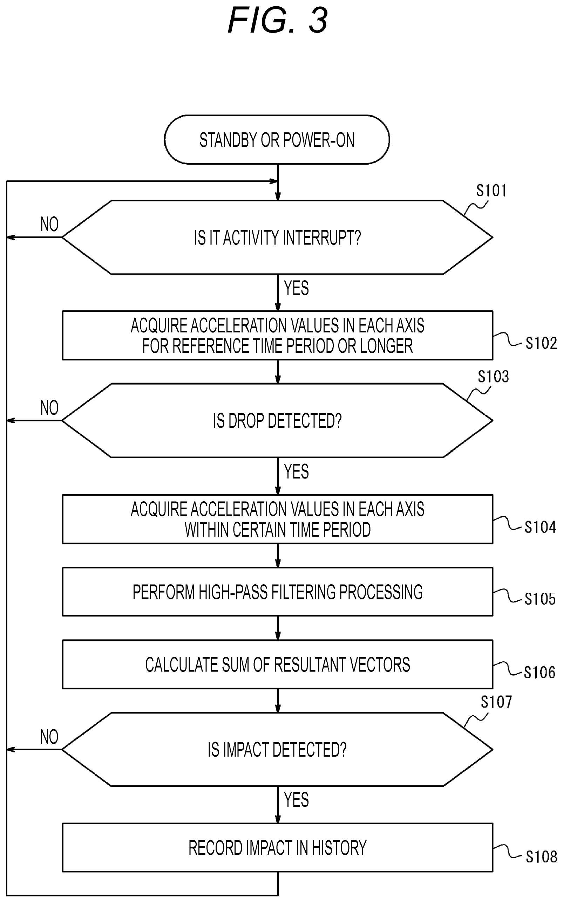

The operation of the impact detection device 20 according to this embodiment will now be described with reference to Fig. 3. The operation to be described below corresponds to an impact detection method according to this embodiment. That is, the impact detection method according to this embodiment includes steps S101 to S108 illustrated in Fig. 3.

When the acceleration sensor 11 interrupts the impact detection device 20 while the electronic device 10 is in a standby state, the electronic device 10 is switched to the power-on state, and step S101 is started. When the acceleration sensor 11 interrupts the impact detection device 20 while the electronic device 10 is in the power-on state, step S101 is started as it is.

In S101, the control unit 21 determines whether an activity interrupt has been input from the acceleration sensor 11. The activity interrupt is a signal indicating that there has been a measurement of acceleration having a magnitude exceeding an activity reference. For example, the activity interrupt is a signal input from the acceleration sensor 11 to the impact detection device 20, the signal being input when an acceleration in any one of the three axes, that is, the X axis, the Y axis, and the Z axis has exceeded ±4 G. That is, the "activity reference" is 4 G, for example. The "activity reference" may be changed as appropriate.

If it is determined that the activity interrupt has been input in S101, step S102 is executed. If it is determined that no activity interrupt has been input in S101, step S101 is executed again.

In S102, the control unit 21 acquires acceleration values corresponding to each axis for a reference time period or longer. Specifically, the control unit 21 receives time-series signals of three-axis accelerations for the reference time period or longer, from the acceleration sensor 11 via the communication unit 23. For example, the time-series signals of three-axis accelerations for the reference time period or longer are signals representing 170 samples corresponding to each of the axes, the samples having been stored in an internal FIFO memory in the acceleration sensor 11 prior to the activity interrupt. "FIFO" is an abbreviation for first in, first out. The "reference time period" is, for example, 360 milliseconds. 144 samples out of 170 samples correspond to samples for 360 milliseconds. The "reference time period" may be changed as appropriate.

In S103, the control unit 21 determines whether a drop of the electronic device 10 from a predetermined height or higher has been detected. Specifically, the control unit 21 determines whether the time-series signals of three-axis accelerations, received in S102, are drop signals indicating that the electronic device 10 has dropped. A drop signal is a signal in which the acceleration in each of the axes keeps taking a value below 1 G and within a certain range around 0 G for the reference time period or more. For example, the drop signal is a signal that keeps satisfying the following conditions over 144 samples: -200 mG < (X-axis acceleration - X-axis adjustment value) < 200 mG, -300 mG < (Y-axis acceleration - Y-axis adjustment value) < 300 mG, and -400 mG < (Z-axis acceleration - Z-axis adjustment value) < 400 mG. That is, for example, the "certain range" for each axis is -200 mG < (X-axis acceleration - X-axis adjustment value) < 200 mG, -300 mG < (Y-axis acceleration - Y-axis adjustment value) < 300 mG, and -400 mG < (Z-axis acceleration - Z-axis adjustment value) < 400 mG. In this example, this "certain range" is set so as to detect a free fall corresponding to 1 meter or more. The "certain range" may be changed as appropriate. In order to determine a free fall corresponding to 1 meter or more accurately, for example, determination may be performed in two stages, using a threshold corresponding to the height of 0.5 meters in combination. In this case, a drop from a height of 0.5 meters or lower is not determined as a drop.

If it is determined that a drop of the electronic device 10 has been detected in S103, step S104 is executed. If it is determined that a drop of the electronic device 10 has not been detected in S103, step S101 is executed again.

In S104, the control unit 21 acquires acceleration values in each of the axes within a certain time period. Specifically, the control unit 21 receives time-series signals of three-axis accelerations from the acceleration sensor 11 via the communication unit 23, within a certain time period following the reception of the drop signal, as a notification signal. For example, the time-series signals of three-axis accelerations within the certain time period are signals presenting eighty acceleration values for each of the axes, each acceleration value being acquired from the acceleration sensor 11 at a cycle of 10 milliseconds. That is, the "certain time period", which is the period for which the acceleration values in each of the axes are acquired, is 800 milliseconds. The "certain time period" is preferably set as a short time period so as to suppress the power consumption, and corresponds to a period of time from a first time point at which recording can be started to a second time point by which acceleration value data can be acquired without shortage. For example, the first time point is a time point of 200 milliseconds from the occurrence of an activity interrupt. Because the "certain time period" is 800 milliseconds, the second time point is a time point of one second from the occurrence of the activity interrupt. The second time point is preferably within a range from 500 milliseconds to 3 seconds allowing fluctuation in the acceleration values to stabilize. That is, the "certain time period" is preferably 300 milliseconds or more and 2800 milliseconds or less. More preferably, the "certain time period" is set to 500 milliseconds or more and 1 second or less. The "certain time period" and the "cycle" may be changed as appropriate.

In S105, the control unit 21 performs high-pass filtering processing. Specifically, the control unit 21 performs high-pass filtering processing on the time-series signals received in S104. For example, the control unit 21 performs high-pass filtering processing by calculating y[k] = A·y[k-1] + A·(x[k] - x[k-1]), where y denotes the acceleration after the filtering processing, x denotes a raw acceleration value, A denotes an attenuation coefficient of 0.1, k denotes current sample data, and k-1 denotes one previous sample data. For the first sample, y[k-1] and x[k-1] do not exist in the first sample, so that y[k-1]=0 and x[k-1]=0 are used. The "attenuation coefficient" may be changed as appropriate.

In S106, the control unit 21 calculates the sum of resultant vectors of the three-axis accelerations. Specifically, the control unit 21 calculates the sum of resultant vectors of the three-axis accelerations having been applied with the high-pass filtering processing in S105.

In S107, the control unit 21 determines whether an impact at a level requiring a notification has been detected. Specifically, when the sum of the resultant vectors calculated in S106 is equal to or greater than a threshold, the control unit 21 determines that the electronic device 10 has received an impact resulting from an unpackaged drop from a specific height or higher. By contrast, if the sum of the resultant vectors calculated in S106 is smaller than the threshold, the control unit 21 determines that the electronic device 10 has not received an impact resulting from an unpackaged drop from a specific height or higher. For example, the "threshold" is 8500 mG. In this example, the "threshold" is set such that an impact is detected when the unpackaged electronic device 10 has dropped from a height of 1 meter or higher, and such that no impact is detected when the electronic device 10 with some packaging has dropped from a height of 1 meter or lower. The impact detection ratio at the time when the unpackaged electronic device 10 drops from a height of 1 meter or higher is preferably 50% or higher. The impact detection ratio at the time when the unpackaged electronic device 10 drops from a height of 0.5 meters or lower is preferably 10% or lower. This "threshold" may be changed as appropriate. It is also possible to set a threshold for determining that no impact is detected even if the unpackaged electronic device 10 drops from a height of 0.5 meters or lower.

If it is determined in S107 that an impact requiring notification has been detected, step S108 is executed. If it is determined in S107 that an impact requiring notification has not been detected yet, step S101 is executed again.

In S108, the control unit 21 records the impact in the history. Specifically, the control unit 21 records the detected impact in the storage unit 22 or an external storage accessible via the communication unit 23. For example, the control unit 21 stores data indicating that the unpackaged electronic device 10 has dropped by 1 meter or more and the date and the time of the drop in the storage unit 22, or stores the data in an external storage system via the communication unit 23. The control unit 21 may cause the display 12 to display a message indicating that the unpackaged electronic device 10 has dropped by 1 meter or more or a message suggesting maintenance of parts of the electronic device 10, may output a voice from the speaker 13, or may transmit the message to the user terminal via the communication unit 23. That is, upon determining that the electronic device 10 has received the impact requiring a notification, the control unit 21 may notify the user that the unpackaged electronic device 10 has dropped from a specific height or higher.

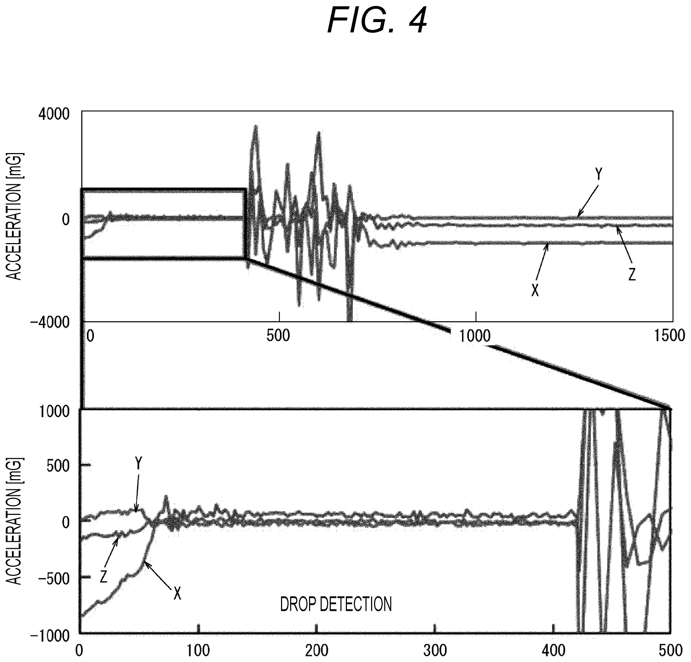

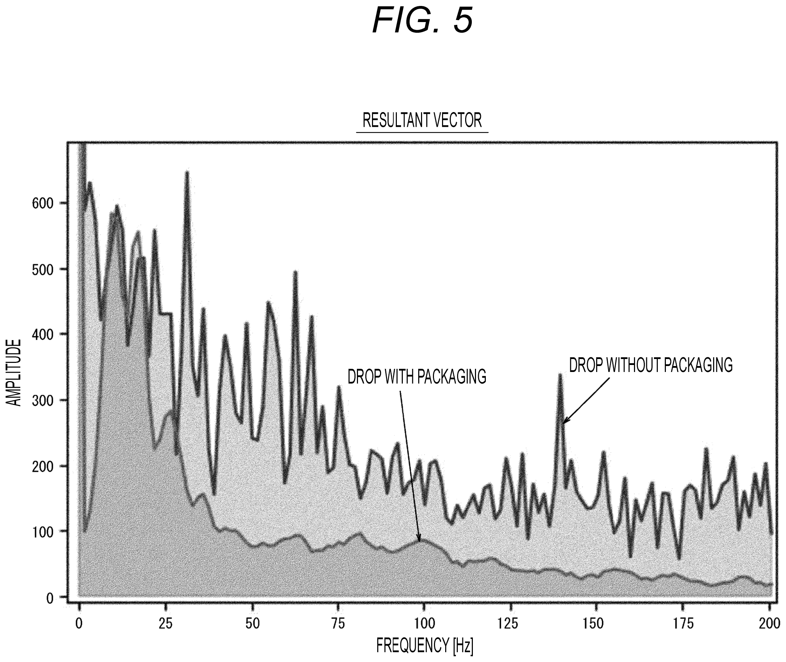

As illustrated in Fig. 4, when the electronic device 10 drops, one instance of high impact occurs, and then a plurality of low impact instances follow until the electronic device 10 stabilizes. The inventors have found that there are differences in the frequency characteristics in impacts between when an unpackaged electronic device 10 has dropped and when the packaged electronic device 10 has dropped, as illustrated in Fig. 5. On the basis of this finding, the impact detection device 20 according to this embodiment is configured to determine whether a drop of the electronic device 10 is an unpackaged drop or a packaged drop, from such differences in the frequency characteristics. Because a cardboard box, a foamed polystyrene box, or a cardboard box containing foamed polystyrene as a packing material has an elastic modulus smaller than the elastic modulus of an unpackaged pump, the duration of impact becomes extended, and low-frequency components become significant. Because a packing box has higher plasticity and higher energy absorption rate, the packing box tends to stabilize immediately after falling.

In this embodiment, when the three-axis accelerations remain near 0 G over a certain time period, it is determined that the electronic device 10 has dropped by 1 meter or more. Then, by using the fact that the frequency characteristics of the acceleration sensor 11 change depending on the rigidity or the rigidity modulus of the object having received the impact, it is possible to make a distinction between impact resulting from a packaged drop and impact resulting from an unpackaged drop. For example, Fig. 5 illustrates frequency spectrums resulting from a packaged drop and an unpackaged drop of the electronic device 10 from a height of 1 meter. The horizontal axis represents frequency, and the vertical axis represents spectral intensity. From this graph, it can be seen that the spectrum of an unpackaged drop of the electronic device, that is, a drop of the bare electronic device has characterizing features in high frequencies, as compared with a packaged drop.

According to this embodiment, a drop and the impact can be detected using a low G acceleration sensor. That is, the function of a high G acceleration sensor can be achieved only with the use of a low G acceleration sensor. As a result, costs can be reduced. The compliance to RoHS can therefore be achieved more easily.

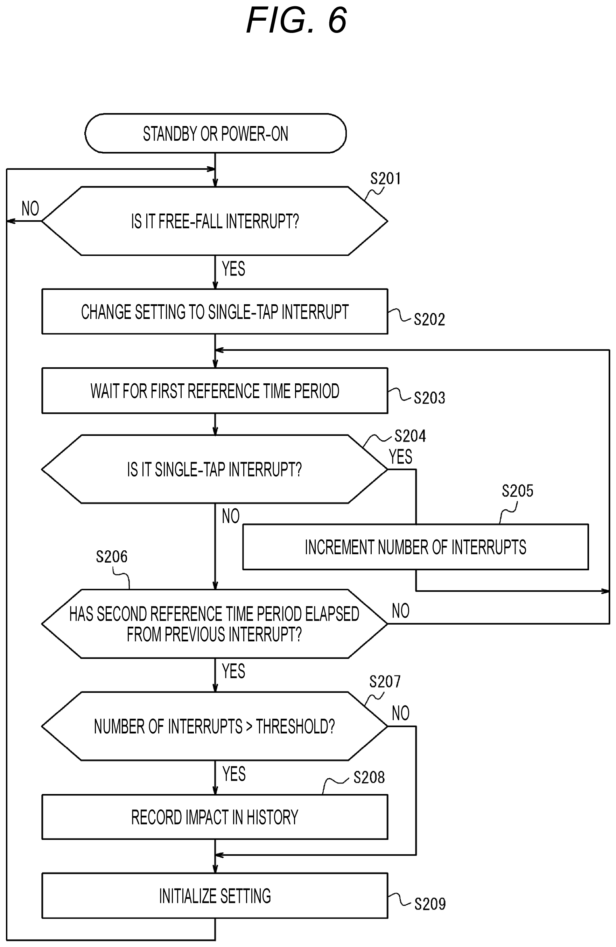

A modification of the operation illustrated in Fig. 3 will be described with reference to Fig. 6.

When the acceleration sensor 11 interrupts the impact detection device 20 while the electronic device 10 is in a standby state, the electronic device 10 is switched to the power-on state, and step S201 is started. When the acceleration sensor 11 interrupts the impact detection device 20 while the electronic device 10 is in the power-on state, step S201 is started as it is.

In S201, the control unit 21 determines whether a free-fall interrupt has been input from the acceleration sensor 11. The free-fall interrupt corresponds to a drop signal indicating that the electronic device 10 has dropped. That is, the control unit 21 determines whether a drop signal has been received from the acceleration sensor 11 via the communication unit 23. The free-fall interrupt is, for example, a signal input from the acceleration sensor 11 to the impact detection device 20 when the accelerations in all of the three axes, that is, the X-axis, the Y-axis, and the Z-axis, remain less than ±312.5 mG for 370 milliseconds. In this example, a condition for an input of the free-fall interrupt is defined so as to detect a free fall of 1 meter or more. This condition may be changed as appropriate.

If it is determined in S201 that a free-fall interrupt has been input, step S202 is executed. If it is determined that no free-fall interrupt has been input in S201, step S201 is executed again.

In S202, the control unit 21 changes the setting to a single-tap interrupt.

In S203, the control unit 21 waits for a first reference time period. The "first reference time period" is, for example, 10 milliseconds. The "first reference time period" may be changed as appropriate.

In S204, the control unit 21 determines whether a single-tap interrupt has been input from the acceleration sensor 11, as the notification signal. The single-tap interrupt is a signal indicating that an acceleration having a magnitude exceeding a single-tap reference has been measured. For example, the single-tap interrupt is a signal input from the acceleration sensor 11 to the impact detection device 20, the signal being input when an acceleration in any one of the three axes, that is, the X axis, the Y axis, and the Z axis has exceeded ±8 G. That is, for example, the "single-tap reference" is 8 G. The "single-tap reference" may be changed as appropriate.

If it is determined that a single-tap interrupt has been input in S204, step S205 is executed. If it is determined that no single-tap interrupt has been input in S204, step S206 is executed.

In S205, the control unit 21 increments a counter for counting the number of interrupts. Step S203 is then executed again.

In S206, the control unit 21 determines whether a second reference time period has elapsed from the previous interrupt. The "second reference time period" is, for example, 500 milliseconds. The "second reference time period" may be changed as appropriate.

If it is determined in S206 that the second reference time period has elapsed, step S207 is executed. If it is determined that the second reference time period has not elapsed yet in S206, step S203 is executed again.

In S207, the control unit 21 determines whether an impact at a level requiring a notification has been detected. Specifically, if the value of the counter for the number of interrupts exceeds a threshold within a certain time period following the reception of a drop signal, the control unit 21 determines that the electronic device 10 has received an impact resulting from an unpackaged drop from a specific height or higher. By contrast, if the value of the counter for the number of interrupts does not exceed the threshold within the certain time period following the reception of a drop signal, the control unit 21 determines that the electronic device 10 has not received an impact resulting from an unpackaged drop from a specific height or higher. The "certain time period" corresponds to the "second reference time period". That is, the "certain time period" is, for example, 500 milliseconds. The "certain time period" is preferably set as a short time period so as to suppress the power consumption, and corresponds to a period of time from the first time point at which recording can be started to a second time point subsequent thereto. The "threshold" of the number of interrupts is, for example, five when the electronic device 10 is in the standby state before step S201 is started, and eight when the electronic device 10 is in the power-on state before step S201 is started. In this example, the "threshold" is set such that an impact is detected when the unpackaged electronic device 10 has dropped from a height of 1 meter or higher, and such that no impact is detected when the electronic device 10 with some packaging has dropped from a height of 1 meter or lower. The impact detection ratio at the time when the unpackaged electronic device 10 drops from a height of 1 meter or higher is preferably 50% or higher. The impact detection ratio at the time when the unpackaged electronic device 10 drops from a height of 0.5 meters or lower is preferably 10% or lower. The "certain time period" and the "threshold" may be changed as appropriate.

If it is determined in S207 that an impact requiring notification has been detected, step S208 is executed. If it is determined in S207 that an impact requiring notification has not been detected, step S209 is executed.

Because step S208 is the same as step S108 illustrated in Fig. 3, the description thereof is omitted.

In S209, the control unit 21 initializes the setting. Then, step S201 is executed again.

As described above, the control unit 21 may increment the counter every time the single-tap interrupt is received as the notification signal, and determine whether the electronic device 10 has received an impact requiring a notification on the basis of whether the value of the counter has exceeded the threshold within a certain time period after the free-fall interrupt is received.

The present disclosure is not limited to the embodiment described above. For example, two or more blocks illustrated in the block diagram may be integrated, or one block may be divided. Instead of executing two or more steps described in the flowchart in the chronological order in accordance with the description, the steps may be executed in parallel or in a different order, depending on the processing capability of the device executing each step, or as necessary. Furthermore, modifications may be made without departing from the spirit of the present disclosure.

### Reference Signs List

- 10: electronic device
- 11: acceleration sensor
- 12: display
- 13: speaker
- 20: impact detection device
- 21: control unit
- 22: storage unit
- 23: communication unit

## Claims

1. An impact detection device comprising:
a communication unit that communicates with an acceleration sensor included in an electronic device; and
a control unit that refers to a notification signal, upon receiving a drop signal indicating that the electronic device has dropped, from the acceleration sensor via the communication unit, the notification signal being received from the acceleration sensor via the communication unit within a certain time period following reception of the drop signal, that determines whether the electronic device has received an impact resulting from an unpackaged drop of the electronic device from a specific height or higher, and that performs recording at least upon being determined that the electronic device has received the impact.

2. The impact detection device according to claim 1, wherein the control unit receives time-series signals of three-axis accelerations, as the notification signal, applies high-pass filtering processing to the time-series signals, and determines whether the electronic device has received the impact based on whether a sum of resultant vectors of the three-axis accelerations after the high-pass filtering processing is equal to or greater than a threshold.

3. The impact detection device according to claim 1, wherein the control unit increments a counter every time a signal indicating that an acceleration of a magnitude exceeding a reference is measured is received as the notification signal, and determines whether the electronic device has received the impact based on whether a value of the counter has exceeded a threshold within the certain time period.

4. The impact detection device according to claim 1, wherein the drop signal is a time-series signal of three-axis acceleration, and is a signal in which the acceleration in each axis continuously takes a value within a certain range less than 1 G and around 0 G over a reference time period or longer.

5. The impact detection device according to claim 1, wherein, when the control unit determines that the electronic device has received the impact, the control unit notifies a user of presence of an unpackaged drop of the electronic device from the specific height or higher.

6. An electronic device comprising:
the impact detection device according to any one of claims 1 to 5; and
the acceleration sensor.

7. The electronic device according to claim 6, wherein the electronic device is a medical device.

8. An impact detection method in which a computer that communicates with an acceleration sensor provided in an electronic device executes:
referring to a notification signal, upon receiving a drop signal indicating that the electronic device has dropped, from the acceleration sensor, the notification signal being received from the acceleration sensor within a certain time period following reception of the drop signal, and determining whether the electronic device has received an impact resulting from an unpackaged drop of the electronic device from a specific height or higher; and
performing recording at least in a case where the computer determines that the electronic device has received the impact.

9. An impact detection program for causing a computer that communicates with an acceleration sensor provided in an electronic device to execute an operation comprising:
referring to a notification signal, upon receiving a drop signal indicating that the electronic device has dropped, from the acceleration sensor, the notification signal being received from the acceleration sensor within a certain time period following reception of the drop signal, and determining whether the electronic device has received an impact resulting from an unpackaged drop of the electronic device from a specific height or higher; and
performing recording at least upon being determined that the electronic device has received the impact.
